# EUROPEAN PATENT APPLICATION

(11) **EP 1 586 334 A1**
(43) Date of publication of application: **19.10.2005**
(21) Application number: 04076161.1
(22) Date of filing: 15.04.2004
(51) Int. Cl.: A61K 47/48

(54) **G-CSF conjugates with peg**

(71) Applicant: TRASTEC scpa, 35127 Padova (IT)
(72) Inventor: Veronese, Francesco M., 35128 Padova (IT); Berna, Manuela, 31050 Pieve di Soligo Treviso (IT)

(57) **Abstract**

The present application relates to novel PEG-G-CSF conjugates in which a PEG molecule is linked to the cysteine residue in position 17 of native G-CSF primary sequence or to the cysteine residue of the corresponding position of a G-CSF analogue.

The present application also describes a process for the manufacture of such conjugates, such process comprising the following steps:
i) subjecting the G-CSF protein to conditions inducing reversible denaturation of the protein,
ii) conjugation of the denatured protein obtained in step i with a thiol - reactive PEG under denaturing conditions,
iii) subjecting the conjugates obtained in step ii to conditions promoting renaturation of the conjugate yielding biologically active G-CSF-PEG conjugate.

## Description

The present invention relates to a site-specific chemical modification of granulocyte colony-stimulating factor (G-CSF).

### BACKGROUND OF THE INVENTION

Granulocyte-colony stimulating factor (G-CSF), the major regulator of granulopoiesis in vivo, represents now a pharmaceutically active protein that stimulates proliferation, differentiation and survival of cells of the granulocyte lineage. Human G-CSF is a glycoprotein of about 20 kDa in size produced by macrophages and stromal cells in bone marrow (Fibbe et al, 1989 Interleukin 1 and poly(rI).poly(rC) induce production of granulocyte CSF, macrophage CSF, and granulocyte-macrophage CSF by human endothelial cells, *Exp Hematol.,* Mar 17(3), 229-34), that was first purified from the conditioned medium of a human bladder carcinoma cell line denominate 5637 (Welte et al., 1985, Purification and biochemical characterization of human pluripotent hematopoietic colony-stimulating factor, *Proc. Natl. Acad. Sci. USA* 82, 1526-1530). The determination of DNA sequence encoding human G-CSF (Japanese Patent Application Laying Open KOHYO No. 500636/88) has enabled the production of human G-CSF by means of recombinant genetic techniques. *E*. *coli* expressed G-CSF differs from the natural material in its lack of glycosylation and the N-terminal methionyl residue incident to bacterial expression (Lu et al., 1989, Disulfide and secondary structure of recombinant human granulocyte colony-stimulating factor, *Arch. Biochem. Biophys* 268, 81-92).

Clinical use of G-CSF (reviewed in Nemunaitis J., 1997, A comparative review of colony-stimulating factor, *Drugs* 54, 709-729; Welte K. et al., 1996, Filgrastim (r-metHuG-CSF): The first 10 years, *Blood* 88, 1907-1929), started in 1986 with the first clinical trials in cancer patients treated with chemotherapy (Bronchud M. H. et al, 1987, Phase I/II study of recombinant human granulocyte colony-stimulating factor in patients receiving intensive chemotherapy for small cell lung cancer, *Br*. *J. Cancer* 56, 809-813) and is now widely used to treat neutropenia. Neutropenia occurs in a wide variety of disease setting, including congenital defects, bone marrow suppression following pharmacological manipulation, and infection. This condition also occurs in cancer patients undergoing cytotoxic chemotherapy. The neutropenia can, in turn, lead to bacterial and secondary infections often requiring hospitalisation. G-CSF can decrease the period of neutropenia or prevent it altogether. In 1991, the United States Food and Drug Administration approved Neopogen (Filgrastin, rh-methuG-CSF) for use by those patients suffering from neutropenia during or after chemotherapy. Treatment with G-CSF can enable a higher-dose-intensity schedule which may allow better antitumor effects (Morstyn D. and Dexter T. M., 1994, Neupogen (r-metHuG-CSF) in Clinical Practice, Marcel Dekker, New York). It was later approved worldwide for use in bone marrow transplantation and more recently for treatment of severe chronic congenital neutropenia. Neopogen has a potential application in mobilising peripheral blood progenitor cells for transplantation (Herman et al., 1996, Characterisation, formulation, and stability of Neupogen (Filgrastim), a recombinant human granulocyte-colony stimulating factor., *Pharm Biotechnol.* 9, 303-28).

More than 80 polypeptide drugs are marketed in United States, and 350 more are undergoing clinical trials. About a third of drugs candidates in clinical trials are polypeptides, but these drugs have normally short in vivo half-life. Many factors are involved in the removal of peptides from the circulation, as proteolytic degradation, renal filtration, and immunogenic and antigenic reactions. In addition, most polypeptide drugs must be delivered by injection, either subcutaneously or intravenously, and the normal low solubility may also be a problem.

To overcome these shortcomings, some methods have been proposed, such as altering peptides amino-acid sequences to reduce degradation by enzymes and antigenic side effects, or fusing them to immunoglobulines or albumin to improve half-life, and incorporating them into delivery-vehicles such as liposomes. For what G-CSF is concerned many patent applications were filed exploiting such methods and papers were also published. Among those reporting variation in the primary sequence we may cite:

U.S. Pat. No. 5,214,132 reports a genetic variant of human G-CSF which differs from native rhG-CSF at position 1, 3, 4, 5 and 17, where instead of the native G-CSF amino acids, the mutein has instead Ala, Thr, Tyr, Arg and Ser respectively (see also, Kuga, et al., 1989, Mutagenesis of human granulocyte colony stimulating factor, *Biochem. Biophys. Res. Commun.* 159, 103-111).

M. Okabe, et al. (In vitro and in vivo hematopoietic effect of mutant human granulocyte colony-stimulating factor, 1990, *Blood* 75(9) May 1, 1788-1793) reported a derivative of rhG-CSF, in which amino acids were replaced at five position at N-terminal region of rhG-CSF, which showed higher specific activity than intact G-CSF in mouse and/or human bone marrow progenitor cells in two assays.

U.S. Pat. No. 5,218,092 discloses a genetic variant of human G-CSF which differ from native rhG-CSF at position 1, 3, 4, 5, 17, 145 and 147 where instead of the native G-CSF amino acids, the mutein has instead Ala, Thr, Tyr, Arg, Ser, Asn and Ser respectively.

WO 01/04329-A reports a rhG-CSF mutein which differ from native rhG-CSF at position 1, 2, 3 or 17.

WO 02/20766-A and 02/20767-A disclose compositions of G-CSF analogues substituted by histidine.

WO 02/077034-A reports modified G-CSF with reduced immunogeicity, obtained by remouving one or more T-cell epitopes, or by reducing the number of MHC allotypes.

An alternative methods for decreasing the clearance rate, improving the stability or abolishing the antigenicity of proteins, is the PEGylation, wherein the proteins are chemically modified by using poly(ethylene glycol) chains. When poly(ethylene glycol) (PEG) is properly linked to a polypeptide, it modifies many of its features while the main biological functions, such as enzymatic activity or receptor recognition, may be maintained. PEG conjugation masks the protein's surface and increases the molecular size of the polypeptide, thus reducing its renal ultrafiltration, preventing to approach of antibodies or antigen processing cells and reducing the degradation by proteolytic enzymes. Finally, PEG conveys to molecules its physicochemical properties and thefore modifies also biodistribution and solubility of peptide and non-peptide drugs. For a review general methods and results of PEGylation see among others reports the following publication and patents:
Davis et al., U. S. Pat. No. 4,179,337, Non immunogenic polypeptide, 1977, which can be considered the basic;
Delgado C. et al., 1992, The uses and properties of PEG-linked proteins, *Crit. Rew. Ther. Drug Car. Sys,* 9 (3, 4), 249-304;
Zalipsky S., 1995, *Ad. Drug Del. Rev.:* Chemistry of polyethylene glycol conjugates with biologically active molecules 16, 157-182, where a collection of methods and prominent reports are described;
F. M. Veronese, 2002, Peptide and Protein PEGylation: a review of problems and solutions, *Biomaterials,* 1-13; F. M. Veronese and J. M. Harris edrs., Ad. Drug Del. Rev., Theme issue on "Peptide and Protein Pegylation I", 2002, 54, 453-606, where an additional collection of paper are reported;
Harris J. M. and Veronese F. M. edrs., Ad. Drug Del. Rev., Theme issue on "Peptide and Protein pegylation II - clinical evaluation", 2003. 55: 1259-1350).

Books were also published on this technique, among others:
Poly(ethylene glycol) Chemistry and Biological Application, J. Milton Harris and
Samuel Zalipsky edrs, 1997, ACS Symposium series 680;
Poly(ethylene glycol) chemistry, Biotechnical and Biomedical Applications, J. M. Harris edr., 1992, Plenum Press;
By now the general benefits enjoyed by pegylated proteins, such as prolonged half-lives or reduced immunogenicity in vivo, are well known. A numbers of studies have been carried out to PEGylate antibodies and antibodies fragments also to reduce the immunogenicity when administered xenogenically.

Several other studies have shown altered biodistribution of antibodies or antibody fragments following PEGylation, leading to greater accumulation in tumours without higher levels in normal tissues as reported by A.P. Chapman in *A.D.D.R.,* 2002, PEGylated antibodies and antibody fragments for improved therapy: a review 54, 531-545).

Schering-Plough has developed a new drug by attaching a 12 kDa mono-methoxy polyethylene glycol to Interferon alpha-2b (Intron A) which fulfils the requirements of a long-acting interferon alpha protein while providing significant clinical benefits (Y. Wang, S. Youngster, M. Grace et al., 2002, Structural and biological characterization of pegylated recombinant interferon alpha-2b and its implications, *A.D.D.R.* 54, 547-570).

In literature it is described also interferon linked on high molecular weight PEG, namely Peginterferon alpha-2a (40 kDa), interferon alpha 2a conjugated to a 40 kDa branched polyethylene glycol moiety, that exhibits sustained absorption and reduced renal clearance , resulting in once weekly instead of a twice-weekly dosing schedule (K. R. Reddy, M. W. Modi, S. Pedder, 2002, Use of peginterferon alpha-2a (40 kDa) (Pegasys) for the treatment of hepatitis C, *A.D.D.R.* 54, 571-586).

U. S. Pat. No. 4,766,106 discloses the increase of solubilization of proteins for pharmaceutical compositions using protein selectively conjugated to a water polymer, selected from polyethylene glycol or polyoxyethylated polyols.

In U. S. Pat. Nos. 5,093,531 and 5,214,131 Sano et al. inventors report a polyethylene glycol derivative capable of modifying the guanidine groups in peptide.

U. S. Pat. No. 5,122,614 discloses a new form of PEG (polyethylene glycol-N-succinimide carbonate) that reacts readily with amino groups of protein in aqueous buffers.

Harris et al. in U. S. Pat. No. 5,252,714 reports the preparation and use of polyethylene glycol propionaldehyde for conjugation to amine groups.

European Pat. applications 0,236,987 and 0,539,167 disclose the use of novel imidate derivatives of PEG and other water-soluble polymers for modifying proteins, peptides and organic compounds with free amino groups.

Several articles and patents are dealing more specifically with G-CSF and its PEGylation, among these Kinstler et al. in U.S. No. 5,985,265 discloses a novel method for N-terminally modifying protein. Using reductive alkylation the end product (protein with an amine linkage to the water soluble polymer) was found to be far more stable the identical polymer-protein conjugate having an amide linkage. In a research paper O. Kinstler et. al describes a site-directed method of joining rhG-CSF to polyethylene glycol (2002, Mono-N-terminal poly(ethylene glycol)-protein conjugates, *A.D.D.R.* 54, 477-485). This selectivity is achieved by conducing the reductive alkylation of proteins with PEG-propionaldehyde at pH 5. As working examples using rhG-CSF and rhMGDF it is demonstrated the application of this methods to improve the delivery characteristics and therapeutic value of these proteins.

WO 90/06952 discloses a chemically modified G-CSF using PEG chains bounded through free amino or carboxyl groups. This PEG-modified G-CSF has prolonged half-life in body, may accelerate the recovery from neutropenia and it has essentially the same biological activity.

WO 00/44785 reports conjugates of rhG-CSF bounded to 1-5 PEG chains, that show improved properties including superior stability, greater solubility, enhanced circulating half-life and plasma residence times.

WO 90/06952 discloses a genetic variant of human G-CSF, prepared according to a method disclosed in Japanese Patent Application Laying Open KOHYO No. 500636/88, chemically modified using PEG, that has essentially the same biological activity, a longer half-life in the body. Furthermore it is observed that this G-CSF-PEG conjugate may accelerate the recovery from neutropenia.

WO 03/006501-A discloses a rhG-CSF that differs from native rhG-CSF in at least one amino acid residue of the amino acid sequence, and with at least one non-polypeptide moiety bounded.

WO 89/06546 discloses a genetic variant of CSF-1 conjugated to polyethylene glycol or polypropylene glycol homopolymers, that retains biological activity and increases circulating mammal half-life.

WO 90/06952 reports a new chemically modified G-CSF bound to polyethylene glycol either through the amino groups of aminoacid residues or through carboxyl groups.

More PEG-G-CSF conjugates having different structures and properties are disclosed in European Pat. No. 0,335,423;

PEGylated G-CSF was also used to investigate different administration rate of the protein, namely the pulmonary delivery. This is reported in R. W. Niven et al., 1994, Pulmonary absorption of polyethylene glycolated recombinant human granulocyte-colony stimulating factor (PEG rhG-CSF), *J. Controlled Release* 32, 177-189.

Finally PEG-G-CSF biological properties are described by Satake-Ishikawa, et al., 1992, Chemical modification of recombinant human granulocyte colony-stimulating factor by polyethylene glycol increases its biological activity in vivo, *Cell Struct Funct.* 17, 157-160.

The G-CSF conjugates mentioned above have been obtained by conjugation via the protein amino-groups. No derivatives have been prepared so far by functionalization of the -SH group, probably because this group is embedded in the tertiary structure of the protein and is very difficult to access.

The present invention has solved this problem and provides a simple method which affords G-CSF-PEG conjugates as a single product with high purity and biologically active.

### DISCLOSURE OF INVENTION

The present invention discloses a way to solve the problems connected with the preparation of suitable G-CSF formulation in view of its therapeutical use. Well known to the expert of the art in the application of proteins for therapy, and of G-CSF among these, is the limitation of short half-life in body, due to the high clearance rate, the proteolytic degradation, and furthermore immunogenic reactions.

The object of the present invention is a new active PEG-G-CSF conjugate, in which one chain of poly(ethylene glycol) (i.e. PEG) is covalently bound through the native protein Cys 17 aminoacidic residue. The G-CSF molecule which can be used for the purposes of the invention may be the native molecule as well as the recombinant one with the N-terminal methionyl residue (Filgrastim) and it may therefore substantially have the following amino acid sequence: 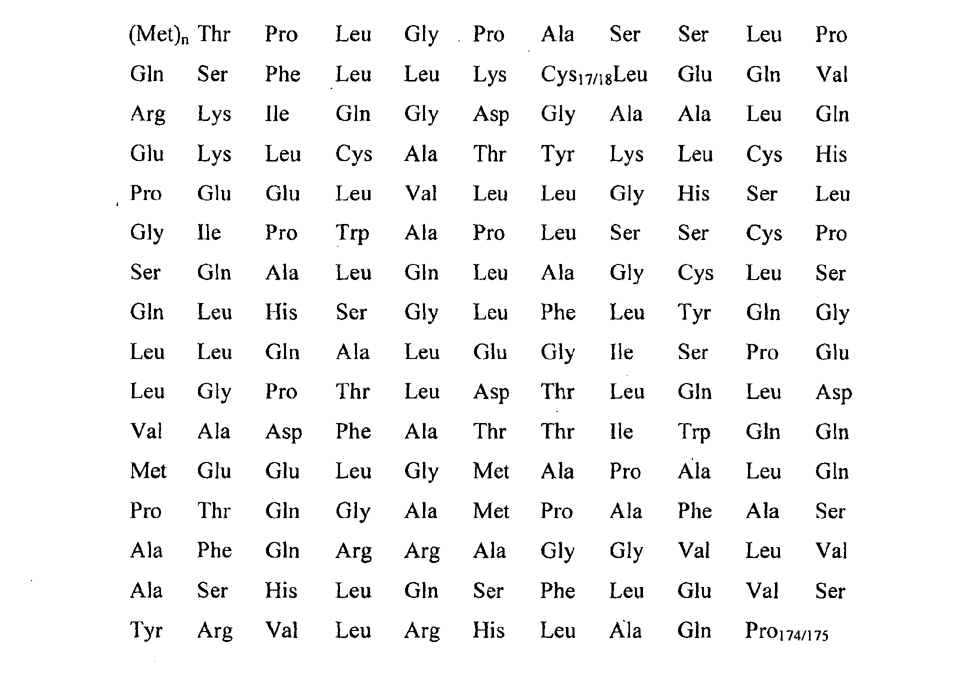 wherein n is 0 or 1.

A further object of the invention is a new process for conjugating PEG to biologically active proteins. It is known to the expert that most applications of PEG conjugation deal with labile molecules, as polypeptides or proteins, consequently the coupling reaction must require mild chemical condition. The most common reactive groups in proteins suitable for polymer conjugation are epsilon amino groups of lysine and alpha amino group of the N-terminal aminoacid. Since either N-terminal amines or lysines in free form are almost always present in any given protein or peptide and G-CSF among these, and since they reacted easily, PEG has been most frequently coupled with proteins through these amino groups. In fact WO 00/44785 discloses G-CSF-PEG conjugates, that have PEG chains bound to lysine or to NH₂ terminal residue. This conjugation is possible for the presence in G-CSF of 4 lysine (Lys 16, Lys 23, Lys 34, Lys 40), one alpha terminal free amino group and for accessibility to the water solvent of all of them. In the reported case the conjugation mixture is represented by a multiplicity of protein species, with PEG linked at different sites, that must be isolated and characterized for a profitable use. To overcome the problem of the multiplicity of conjugates some authors took advantage of the fact that lysine ε-amines are good nucleophyles above pH 8 since their pKa is around 9.5, while the α-amino groups of N-terminal amino acids are less basic than Lys and reactive at lower pH also. The reaction pH could therefore be critical to achieve selectivity in conjugation and consequently simplified mixture of products could be obtained.

In the U. S. Pat. No. 5,985,265 this property was exploited and a chemical method to preferentially modify the N-terminal amino acid residue using a reaction around pH of 5 was disclosed. In this case the reaction was preferentially towards the N-terminal residue, although it was non complete and the lysine amino groups may react as well although at a much lower extent. The obtained mixture must therefore be purified to reach the wanted homogeneity of product.

Another important potentially reactive residue in proteins is the thiol group, that is very reactive towards several thiol reactive PEGs, such as monomethoxy-PEG-maleimide (m-PEG-maleimide), m-PEG-o-pyridyldisulphide, m-PEG-vinylsulfone which are reacted according to methods already known in literature [Veronese F (2001) Peptide and protein PEGylation: a review of problems and solutions. *Biomaterials* **22**, 405-417; Roberts MJ et al. (2002) Chemistry for peptide and protein PEGylation. *Adv.Drug Deliv.Rev.* **54,** 459-476].

Several articles describe specific pegylation on cysteine residue (Cantin AM et al, 2002, Polyethylene glycol conjugation at Cys232 prolongs the half-life of alphal proteinase inhibitor. *Am JRespir Cell Mol Biol.:* 27(6): 659-65; Leong SR et al, 2001, Adapting pharmacokinetic properties of a humanized anti-interleukin-8 antibody for therapeutic applications using site-specific pegylation *Cytokine.* 16(3): 106-19; Collen D et al., 2000, Polyethylene glycol-derivatized cysteine-substitution variants of recombinant staphylokinase for single-bolus treatment of acute myocardial infarction. *Circulation* 102(15): 1766-72; Goodson RJ et al, 1990, Site-directed pegylation of recombinant interleukin-2 at its glycosylation site *Biotechnology* 8(4): 343-6; Paige et al. Prolonged circulation of recombinant human granulocyte-colony stimulating factor by covalent linkage to albumin through a heterobifunctional polyethylene glycol, *Pharmaceutical Research,* Vol. 12, No. 12, 1995).

Unfortunately, cysteine residues are rarely present in proteins in the free form and furthermore they are commonly present as disulphide in cystine and, when free, they are often involved in active region of the molecule.

In rhG-CSF primary sequence there are five Cys, four of them are involved in two disulphide-bound (Cys 36-Cys 42, Cys 64-Cys 74) but luckily one, Cys 17, is free. Arakawa et al., 1993, report that Cys 17 is not accessible to carboxymethylation by a hydrophilic agent, iodo-acetic acid, under native conformation, but it becomes fully reactive when rhG-CSF is denatured. In other experiments, the free sulphydryl has been shown to react very slowly with a hydrophobic modifying agent, dithiobis-nitrobenzoic acid (DTNB) under non-denaturating conditions. From these experiments and from the tridimentional structure as assessed by NMR it is postulated that the free cysteine resides inside an hydrophobic environment of the molecule and as such it is inaccessible to chemical modification by hydrophilic alkylating agents whereas is more accessible to hydrophobic agents. Conformational studies of G-CSF have in fact demonstrated that the -SH at position 17 is in a hydrophobic pocket of the protein, only partially exposed to solvent. In addition Wingfield et al.(1988, Characterization of recombinant-derived granulocyte-colony stimulating factor (G-CSF), *Biochem J.* Nov 15; 256(1), 213-8) found that a Cys to

Ser substitution at position 17 resulted in no appreciable changes in physical or biological properties of the protein.

The inventors have now discovered a method for the manufacture of G-CSF conjugates specifically conjugated at the thiol group of Cys 17.

The method comprises the following steps:
i) subjecting the G-CSF protein to conditions inducing reversible denaturation of the protein,
ii) conjugation of the denatured protein obtained in step (i) with a thiol reactive PEG, under denaturing conditions,
iii) subjecting the conjugate obtained in step (ii) to condition which promote renaturation of the conjugate and afford the desired conjugate.

Biological experiments reported by the inventors show that PEG-G-CSF conjugates prepared according to the above method, maintain biological activity in cell proliferation assay.

The reversible denaturation of step (i) is carried out in the presence of one or more denaturating agents like urea, guanidine chloride or isothiocyanate, dimethyl-urea, high neutral salt concentrations and solvents (such as for example, acetonitrile, alcohols, organic esters, dimethylsolfoxide). For reversible chemical denaturation it is meant a process in which a protein, upon denaturation, loses both its native structure and function, however upon removal of the denaturant, recovers either structure and functional properties. In the presence of such denaturating agent/s the protein is unfolded and the thiol group of Cys-17 is exposed and becomes accessible for functionalisation. The denaturation process can be monitored by far UV-CD spectra. The amount of denaturating agent can be selected by the skilled person depending from the denaturant agent used. For example for the case of Guanidine Chloride a concentration of 4 M was found to be suitable.

The unfolded protein obtained in step (ii) is reacted maintaining denaturating condition with an activated PEG which has specific reactivity for the thiol group "thiol reactive PEG".

Thiol reactive PEGs are known to the skilled person; preferred are: orthopyridyl disulphide, vinylsulfone, N-maleimide, iodoacetamide.

The PEG can be linear or branched, and may have a MW of 800 to 80.000 Da and preferably 5.000 to 40.000 Da.

Bi-functionalized PEGs like OPSS-PEG-OPSS; VS-PEG-VS; VS-PEG-OPSS, MAL-PEG-MAL, MAL-PEG-OPSS, MAL-PEG-VS may also be used.

The completeness of the reaction can be assessed by usual analytical methods, like HPLC or by monitoring the presence of thiol groups by colourimetric tests.

Once step (ii) is completed, the conjugate can be let refold exposing it to renaturating conditions according to standard methods as for instance disclosed in Mechanisms of protein folding, Pain RH (edt.), IRL-Press, Oxford U.K., 1994 and in Middelberg APJ. Preparative protein refolding. Trends Biotechnol.20, 437-443, 2002 (herein incorporated by reference), yielding the original α-helix structure

Typical methods involve removal of the denaturating agents by dialysis, ultrafiltration, chromatografic methods.The pH and the temperature used in steps i, ii, iii can be selected by the skilled person and may very depending from the reactive agents used, the type of activated polymer and from its molecular weight.

The conditions and the reagents have to be selected so that irreversible denaturation or cleavage of the cysteine -S-S- bond of the protein are prevented.

Mild values of pH are preferred for example 4 to 10, more preferred 6 to 8 and even more preferably close to neutrality.

Low temperatures are also preferred, 0 to 30 °C, although for conjugation of high MW PEGs (polymers) higher yields can be obtained if higher temperatures are used (for example 40°C).

For the skilled person it is also clear that the method of conjugation described above can be used to prepare conjugates of any protein comprising an hindered cysteine group.

The first step of the method is preferably carried out at a temperature comprised between 5°C to 50°C , preferably at a temperature of between 20°C to 40°C.

The renaturation may be obtained by removing the denaturant agent by dialysis, ultrafiltration, chromatography or dilution.

The successful conjugation of G-CSF with thiol reactive PEGs, as herein reported, which was carried out with a method that yields to mono-functionalized protein, paves the way to the well known and largely exploited technological and biological advantages of PEGylation.

The proposed method allows to get products with large increase in Mol.Wt. and, more important, in large hydrodinamic volume of the molecule.

As known the hydrodinamic volume of PEG corresponds to about 3-4 fold that expected from its weight and therefore the conjugates with 5000, 10000 and 20000 Da PEG, as described in the examples of this patent, correspond to the volume of a protein of about 40000, 50000 and 60000 Da. Products of this volumes are known to overcome the threshold of kidneys glomerular filtration and consequently a significant increase in blood retention time can be always expected.

The patent application therefore discloses a pharmaceutical composition comprising the conjugates prepared as described above for oral, injectable, nasal, pulmonary or suppository or transdermal applications.

The thiol conjugation procedure reported here, which exploits for the conjugation the reversible denaturation to expose the lone free SH of cysteine number 17 in the sequence, is more convenient than those reported in the prior art and which are based on amino group modification. In fact, in amino group modification, multiple products are commonly obtained also if one increases the specificity of the reaction by lowering pH to target the alpha amino acid residue only. The products obtained in amino modification must afterwards be properly fractionated and purified by time consuming procedures and loss of product.

The here proposed method may be easily scaled up and few steps are necessary to obtain the final product:
i. dissolution in denaturing solution,
ii. addition of the activated PEG in powder form,
iii. removal of the denaturant agent and protein refolding by dialysis or column chromatography.
   Optionally the denaturing agent (i.e. guanidine HCl, urea or other denaturating agents) may be added to an aqueous solution of the protein, concomitantly to the addition of the activated PEG during step ii.
   It is well known that a common problem in protein PEG conjugation relies in loss of catalytic activity in case of enzymes, or of the recognition in case of a ligand modification such as a cytokine. As an typical example stands the several fold reduction in alpha-interferon activity that takes place following conjugation.
   This was not found to be the case of our G-CSF thiol modification. Most probably this positive behaviour is related to the single point attachment achieved by our technology, to the position of the SH in the three dimensional arrangement of the protein far from the recognition site. The maintenance of the original biological activity of our conjugate was assessed in cell culture using NFS-60 cells. These possess over-expressed number of receptors to G-CSF and the grow of the cells is proportional to the cytokine concentration. The G-CSF activity, expressed as EC50, namely the concentration that stimulate the 50% of the maximal grow was very similar to the one of the native protein as it is exemplified in the example reported and in figure n. 12.
   In another aspect of this invention, bi-functionally activated PEG derivatives (OPSS-PEG-OPSS; VS-PEG-VS; VS-PEG-OPSS, MAL-PEG-MAL, MAL-PEG-OPSS, MAL-PEG-VS) may be used to generate dimeric G-CSF (i.e. G-CSF-PEG-G-CSF) in which two G-CSF monomers are linked together at the thiol group of Cys 17 via PEG. The *in vitro* activity of such obtained dimers are comparable to those of the corresponding pegylated form of G-CSF. The main advantage of these dimers is the reduction of the in vivo elimination rate (if compared with the corresponding pegylated form of G-CSF) due to the increasing of their molecular mass.

### Example 1: Preparation of 5 kDa PEG Conjugated to rhG-CSF by direct PEGylation

### Modification of G-CSF with 5 kDa methoxy-PEG-orthopyridyl-disulfide

Methoxy-PEG-orthopyridyl-disulfide (1.81 mg, 0.360 µmol) was added to 0.67mg, 0.0356 µmol of rh G-CSF, in 2 ml of buffer (0.5 M Tris, pH 7.2), molar ratio reagent to rhG-CSF of 10/1. The reaction was left allowed to proceed at room temperature for several hours, to reach constant formation of PEG-G-CSF conjugate as verified by HPLC and SDS-PAGE (see below).

### Characterization of PEG-OPSS G-CSF conjugates

### 1 DTNB colorimetric assay (Ellman)

Ellman's reagent, 5,5'-dithiobis(2nitrobenzoic acid) (DTNB) permits the estimation of free thiols in proteins and other biological systems. The assay sensibility is due to the high molar adsorption coefficient of 2-nitro-5-thiobenzoate anion, which is produced together with the mixed disulfide from the reaction of a thiolate anion with DTNB (Habeeb A. F. S. A., 1972, Reaction of protein sulphydryl group with Ellman's reagent, Methods in Enzymology 25, 457-464). In this way it is possible to verify the percentage of sulphydryl groups in native or conjugate protein, and so to know the modification amount.

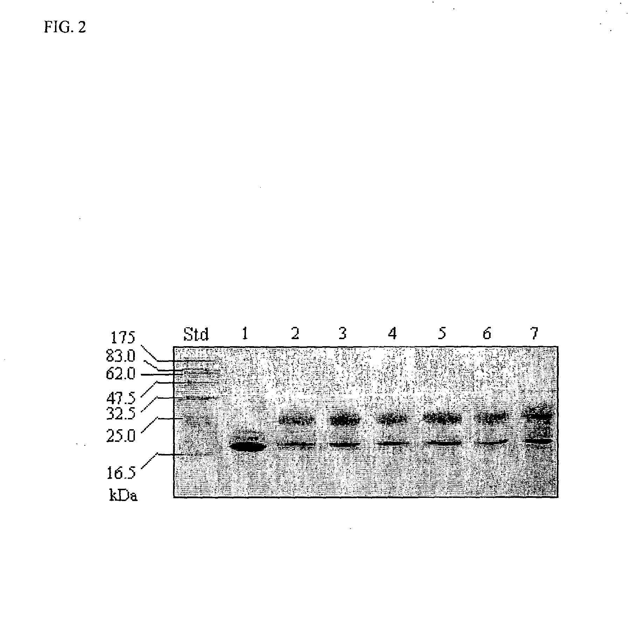

2. SDS-PAGE The amount of rhG-CSF derivatization was estimated qualitatively by SDS-polyacrylamide gel electrophoresis. A gel with a low degree of cross-linking (60% acrylamide, 0.8% bisacrylamide) was employed to allow the conjugate migration. The reaction mixture was analysed at different time. Proteins visualized by Coomassie Blue staining revealed the presence of a small amount of new high molecular weight protein specie, corresponding to a 5 kDa PEG-OPSS chain bound to rhG-CSF together with the major spot of the starting material unreacted protein. See figure 2.

### 3.RP-HPLC C4.

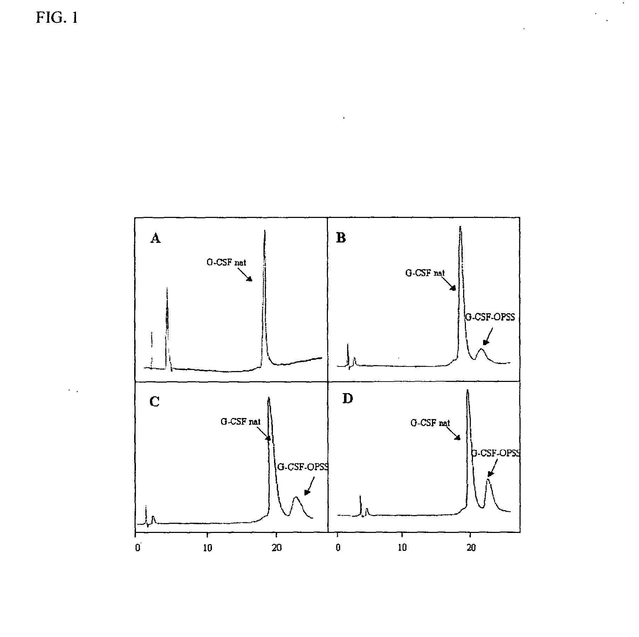

The reaction mixture was analysed by reverse phase HPLC using a C4 Vydac column according to the following elution condition. Eluent A, 0.05% TFA in water, and B, 0.05%TFA in acetonitrile. The following linear gradient was used: 40%B for 4 minutes, up to 70%B in 18 minutes, 95%B in 2 minutes, stay at 95%B for 6 minutes and than 40%B in 2 minutes. The flow was 0.8 ml/min and UV lamp at 226 nm. The time course of the reaction showing the formation of limited amount of conjugate while most of the protein is eluted unmodified. See figure 1.

### Modification of G-CSF with 5 kDa methoxy-PEG-vinylsulfone

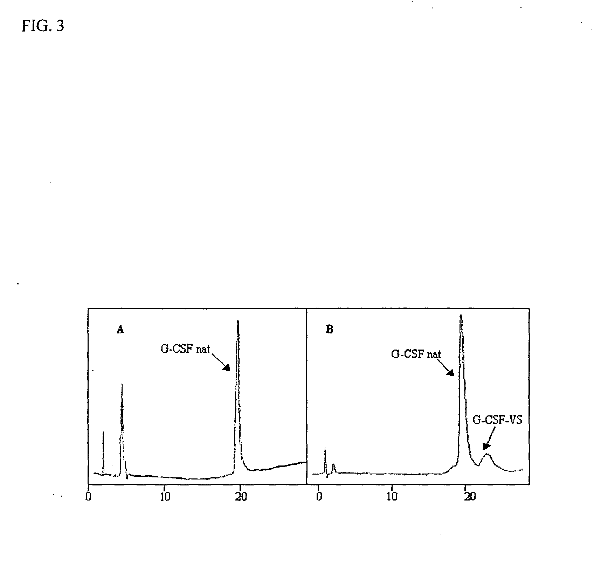

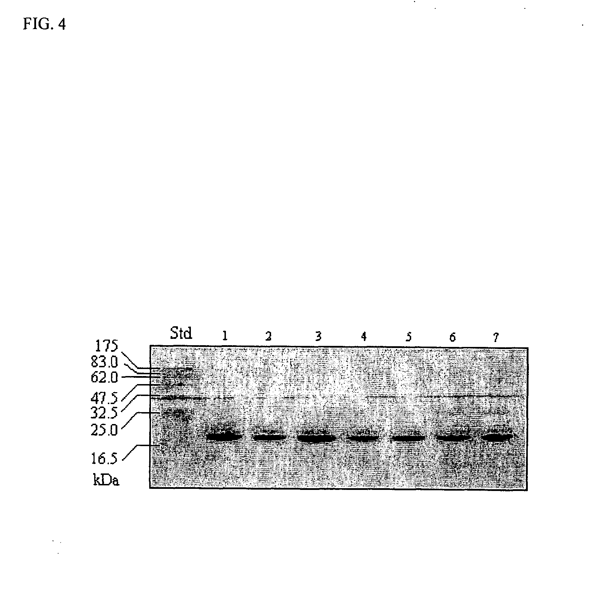

Methoxy-PEG-vinylsulfone (5.29 mg, 1.058 (mol) was added to 2.18 mg, 0.116 (mol of rh G-CSF, in 6 ml of buffer (0.5 M Tris, pH 7.2), molar ratio reagent to rhG-CSF of 10/1. The reaction was left allowed to proceed at room temperature for several hours, to reach constant formation of PEG-G-CSF conjugate as verified by HPLC and SDS-PAGE (see below). In figure 3 or 4 the HPLC profile and SDS-PAGE of the reaction mixture at different times. The figures demonstrate that a conjugation of G-CSF takes place but only to a very limited extent although the reaction was left proceed for several hours.

### Example 2: Reversible denaturation of rhG-CSF

Guanidine HCl 8M in phosphate buffer 0.1 M, pH 7.27 were added at different amount to a rh-GCSF buffer solution in order to reach a final concentration of 2, 4 e 5 Molar. After 4 hours incubation at room temperature the mixture was dialyzed against an aqueous acidic solution, to remove the denaturant and to reach the G-CSF stable condition of pH 3.5.

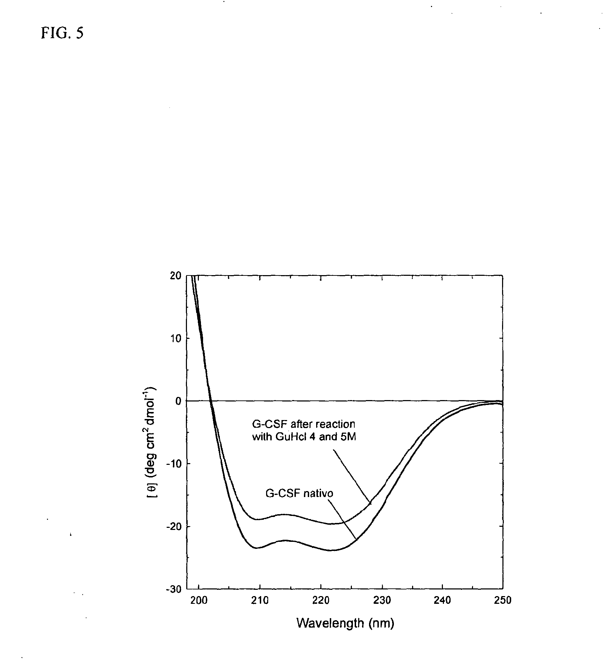

Figure 5 reports the CD spectrum of the starting proteins and after the denaturation - renaturation process. The regain of the secondary structure is evident from the 220-208 nm ellypticity ratio of before and after treatment. The following experiment demonstrates that G-CSF may be subject to denaturation and renaturated to the original structure.

### Example 3: Preparation of 5 kDa PEG rhG-CSF conjugate by thiol reagents in denaturant conditions

### Conjugation of 5 kDa PEG-UPSS and PEG- VS to rhG-CSF.

**3a:** To a G-CSF buffer solution Tris 0.5 M, 6 M Urea, pH 7.2 was added in order to reach a final concentration of 3 M urea. To this solution mPEG-OPSS or mPEG-VS were added at a molar ratio of 10 moles of reagent per mole of protein. The reaction was allowed to proceed at room temperature for 6 hours and stopped by 0.1 N HCl addition.
**3b**: G-CSF was added to a solution of 8 M Guanidine HCl in phosphate buffer 0.1 M, pH 7.27, to have a final denaturant concentration of 2, 4, 6M. The denaturated G-CSF was added to PEG-OPSS or PEG-VS solution in order to reach molar ration of 10 moles of reagents per mole of protein. After 4 hours at room temperature the reaction was stopped with HCl 0.1N.

### Example 4: Characterisation of 5 kDa PEG conjugated to rh-G-CSF

1. RP-HPLC chromatography.
   The reaction mixture was analysed by reverse phase HPLC using a C4 Vydac column according to the following elution condition. Eluent A, 0.05% TFA in water, and B, 0.05%TFA in acetonitrile. The following linear gradient was used: 40%B for 4 minutes, up to 70%B in 18 minutes, 95%B in 2 minutes, stay at 95%B for 6 minutes and than 40%B in 2 minutes. The flow was 0.8 ml/min and UV lamp at 226 nm.

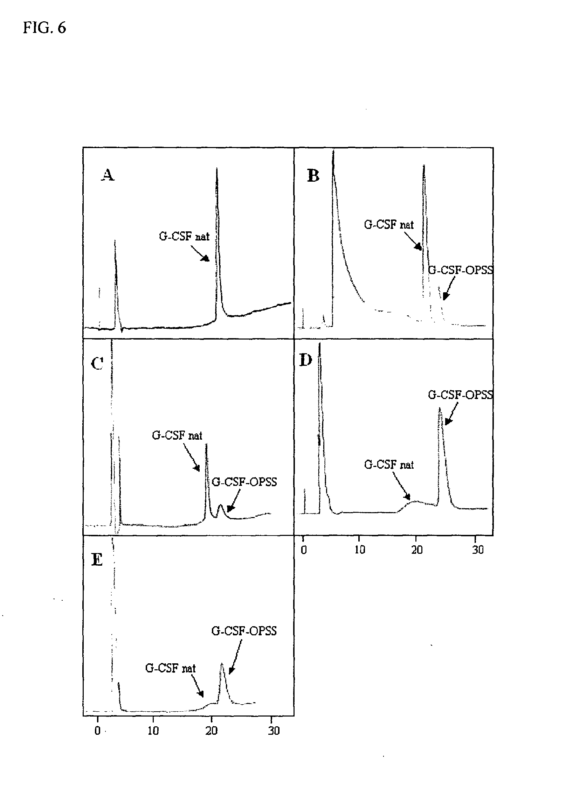

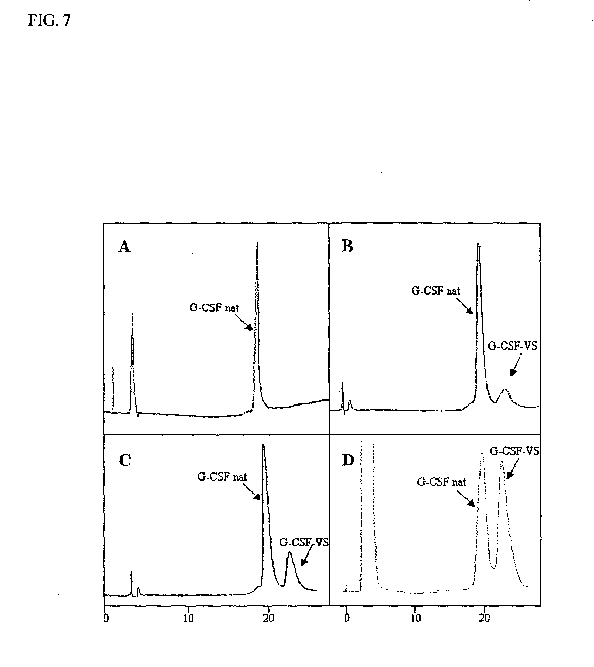

   Figure 6A shows the chromatogram of the native G-CSF. Figure 6B shows the formation of the conjugate (PEG-OPSS) using urea as denaturant. Figures 6C, D, E show the formation of the conjugate (OPSS) in different reaction condition, namely in figure 6C the amount of guanidine HCl was 2 M, in 6D, 4M, and in 6E, 6M. As it possible to see in figure 6C-E the percentage of conjugate is increased following the concentration of guanidine. Figures 7B, C, D show the formation of the coniugate (PEG-VS) respectively under the same conditions (guanidine concentration) of figure 6C, D, E.
   The best condition chosen was 4M of guanidine, because it represent a good compromise between yield modification and mild denaturating condition.
   The time course of the reaction showing the formation of limited amount of conjugate while most of the protein is eluted unmodified.
2. MALDI-TOF mass spectroscopy of G-CSF native and of G-CSF-PEG-OPSS (5kDa) conjugate fractionated from the reaction mixture using HPLC chromatography.
   Figure 8 A (native G-CSF) reports a peak at 18.930 Da, that corresponds to the molecular weight of G-CSF native. Figure 8 B (peghilated G-CSF) reports a peak at 24.711 Da, that corresponds to the molecular weight of G-CSF native bounded to a single chain of PEG-OPSS (5kDa).
   Figure 9 (reaction mixture of G-CSF) reports a peak at 24.583 Da, that corresponds to the molecular weight of G-CSF native bounded to a single chain of PEG-VS (5kDa).
3. Far-ultraviolet Circular Dichroism spectra of G-CSF native and G-CSF-PEG conj ugates.
   CD spectra were recorded on a Jasco Model J-700 spectropolarimeter at 25°C at a protein concentration around 0.1 mg/ml using 0.1 cm path length quartz cell.
   Figure 9A reports far-UV CD spectra of G-CSF native and G-CSF-PEG-OPSS conjugate. These spectra are typical of α-helical polypeptides with ellipticity minima at 208 and 222 nm. A difference of α-helical percentage between native and conjugate protein should due to aggregation problems that can distort protein concentration and so spectra normalisation. Figure 9B reports far-UV CD spectra of G-CSF and G-CSF-PEG-VS conjugate. In this case the secondary structure variation between native and conjugate protein is more evident.
4. Studies of fluorescence emission.

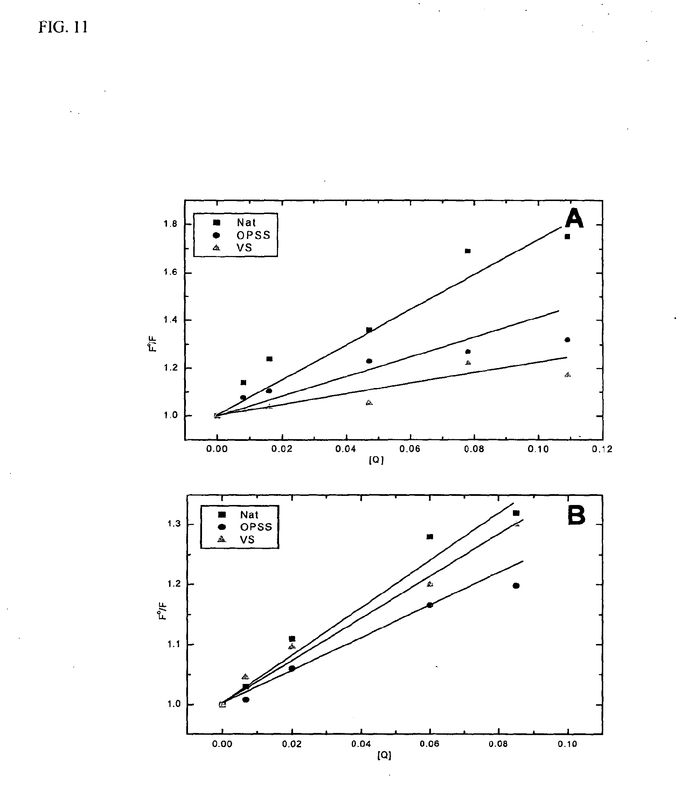

The fluorescent spectrum of G-CSF native and G-CSF-PEG-OPSS and G-CSF-PEG-VS conjugates were recorded at 25°C on a Perkin Elmer LS-50, exciting the samples (1.3 µM) at 295 nm and recording the emission fluorescence in the wavelength range 303-500 nm. Spectra were recorded at the same condition after addition of negative (Nal) and positive (CsCl) quenchers. The fluorescence spectra of native and conjugates differ only for a blue-shift of 5 nm (from 350 to 345 nm) in emission wavelength, too small for indicate a change in fluorofores exposition to solvent. Fluorofores (Trp 59, Trp 119) have a polar around, and so are probably exposed to solvent both in native and in conjugated protein. Figures 11A-B report quenching experiments using CsCl (A) and Nal (B) for G-CSF native, G-CSF-PEG-OPSS and

G-CSF-PEG-VS. These studies reveal a small variation in solvent accessibility of Trp in native and conjugated protein, variation probably due to the presence of a PEG chain around Trp.

### Example 5: Preparation of 10 kDa PEG Conjugated to rhG-CSF by thiol reagent in denaturant condition

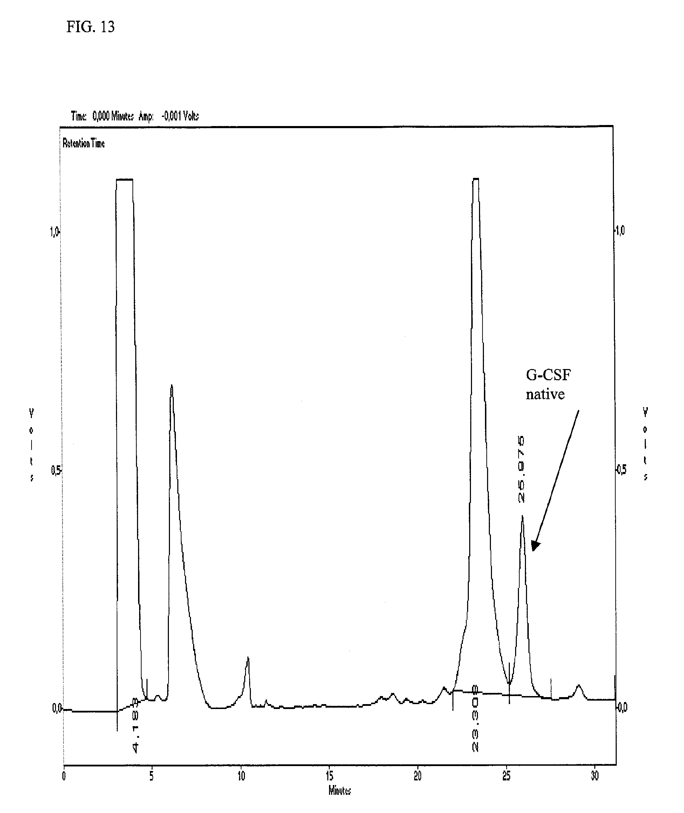

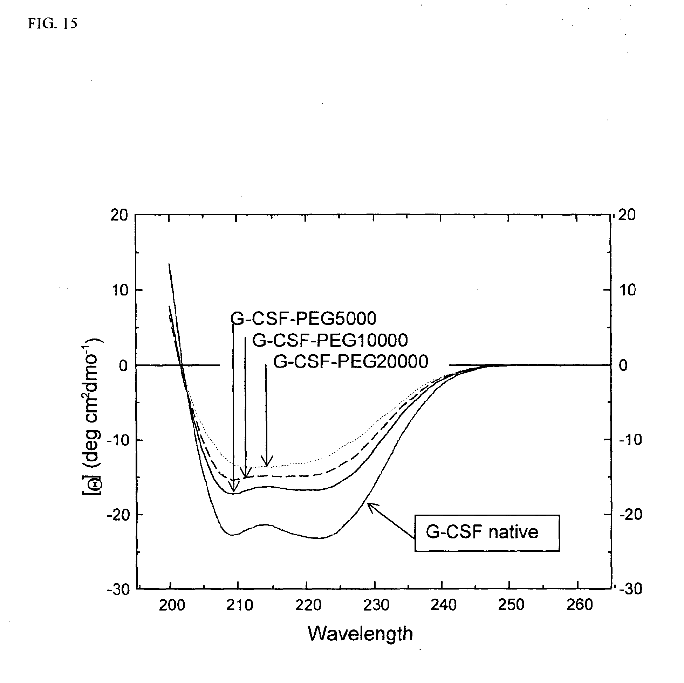

**5a.** 1.5 ml of rhG-CSF (1.46 mg/ml) was added to 1.5 ml of a solution 6 M Guanidine HCl in phosphate buffer, pH 7.2. At this solution was added mPEG-OPSS or mPEG-VS (10 kDa) in a molar ratio of 10 moles of polymer per mole of rhG-CSF. The reaction mixture allowed to proceed at room temperature in argon atmosphere for 20 hours and the reaction was stopped with HCl 0.1M.
5b The characterisation of the conjugates was realized by RP-HPLC chromatography (Figure 13) and far-UV CD spectra (Figure 15). The RP-HPLC was carried out using a C4 Phenomenex column.

### Example 6: Preparation of 20 kDa PEG Conjugated to rhG-CSF by thiol reagent (mPEG-OPSS and mPEG-VS) in denaturant condition

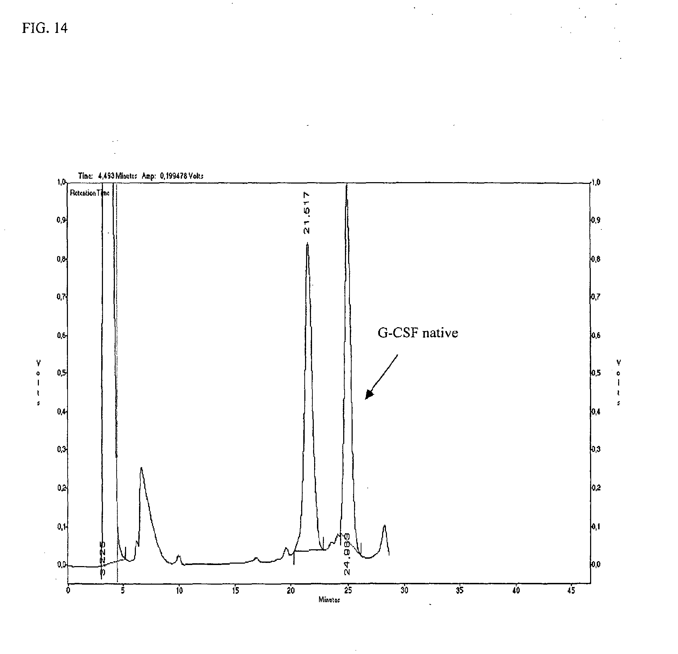

**6a.** 1.5 ml of rhG-CSF (1.46 mg/ml) was added to 1.5 ml of a solution 6 M Guanidine HCl in phosphate buffer, pH 7.2. At this solution was added mPEG-OPSS or mPEG-VS (20 kDa) in a molar ratio of 10 moles of polymer per mole of rhG-CSF.
   The reaction mixture allowed to proceed at 40°C for 1 hour and than at room temperature for other 4 hours. The reaction was stopped with HCl 0.1 M.
**6b** The characterisation of the conjugates was realized by RP-HPLC chromatography (Figure 14) and CD spectra in far UV (Figure 15).

### Example 7: Preparation of 20 kDa PEG Conjugated to rhG-CSF by thiol reagent (mPEG-MAL) in denaturant condition

***Solution A:*** a 3mg/ml rhG-CSF solution buffered to pH7.2 with 0.5M Tris-HCl was prepared (solution A)
***Solution B:*** mPEG-Maleimide was dissolved in a 6M guanidine HCl solution, buffered to pH 7.2 with Tris-HCl (0.5M), in order to obtain a final concentration of 30mg/ml (PEG-Mal).

6 ml of solution A and 6 ml of solution B were mixed and the resulting mixture was allowed to react at room temperature for I hour (molar ratio rhG-CSF/PEG-Mal=1:10). The yield of pegylation was higher than 95%.

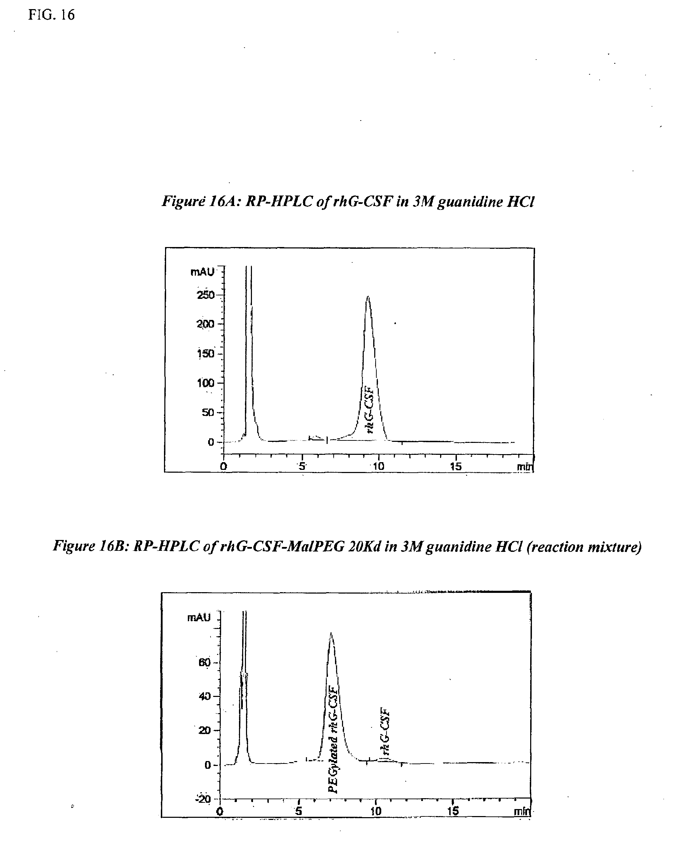

Figure 16 shows the RP-HPLC chromatograms of the native rhG-CSF (fig. 16A) and those of the reaction mixture after 1 hour at room temperature (fig. 16B).

### Example 8: In Vitro cellular proliferative activity in NFS-60 cells.

The biological assay in based on the evaluation of the stimulating effects of rhG-CSF in the proliferation of a sensitive cell line developed from a mouse myeloblastic leukaemia reach of G-CSF receptors: NFS-60 cells. The protein binding to the cells leads to an exponential proliferative reaction and the final cell concentration was found to be proportional to the concentration of G-CSF present in cell culture. This allows to quantify the biological potency of G-CSF sample versus a standard or a reference preparation, applying a dose-reaction ratio. For the test NFS-60 cells are maintained as suspension cultures in RPMI 1640 supplemented with 5% foetal bovine serum, 4 mM Glutamine and rhG-CSF to a final concentration of 18.000 IU/ml of medium. Before of each experiment, the cells are washed three times in ice-cold phosphate-buffered saline (PBS) and suspended again in assay medium (2.5% foetal bovine serum, 4 mM Glutamine) to remove the rhG-CSF contained in growth medium. In our case solution of G-CSF and PEGs conjugates were subjected to a serial dilutions in assay medium, transferred in duplicates (50 µl each) to ninety-six well plates and mixed with an equal volume of the pre-washed cell suspension to a final density of 1.0 x 10⁵ cells per ml. Plates were then incubated at 37°C in CO₂ for 72 hours before addition of 10 µl of MTT solution (100 mg MTT in 20 ml of sterile warm PBS). The reaction was allowed to proceed for 4 hours at 37°C in CO₂ oven.

The microsomal dehydrogenated enzymes of living cells turn the formazan salts MTT ((3[4,5 Dimethylthyazol-2yl]-2,5diphenyltetrasolium bromide) into blue crystals. Tris crystals were solubilized using 100 µl of a solution of SDS 15% in 0.01 N HCl. Absorbance at 570 nm is directly proportional to the number of living cells.

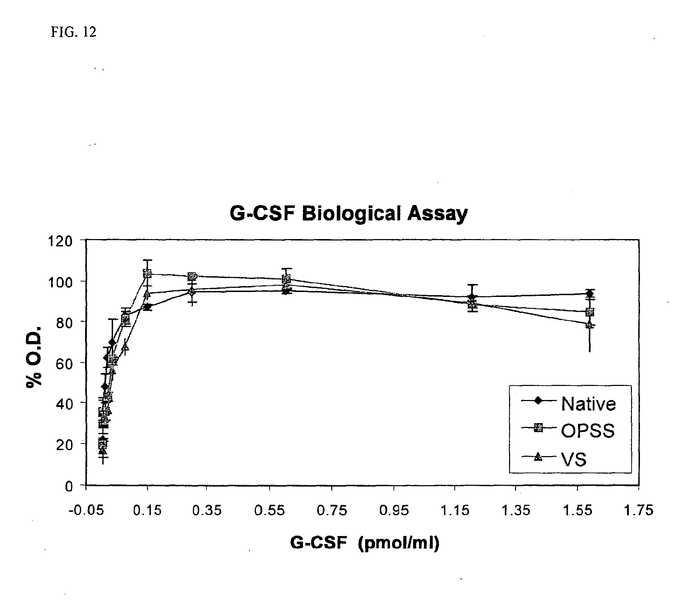

The activities of G-CSF and the conjugates as stimulating factors, calculated as EC₅₀ values from the activity plot (Figure 12), are reported in the following table.

**In vitro activities in NSF-60 cells**

| Sample | EC₅₀ (pmol/ml) | Activity |
|---|---|---|
| rhG-CSF | 0.0096 | Ix |
| rhG-CSF-PEG-OPSS | 0.0145 | 0.66x |
| rhG-cSF-PEG-VS | 0.039 | 0.246x |

EC₅₀ values (pmol/mL) is the amount of rh-G-CSF that promotes the cell growth of 50% against the maximum cell growth. Therefore the lowest is the EC₅₀ value the highest is the rhG-CSF activity.

Taking into account the accuracy of the method, rhG-CSF-PEG-OPSS can be considered almost active as native rhG-CSF.

### DESCRIPTION OF THE DRAWING

Figure 1: RP-HPLC chromatograms. G-CSF native (**A**); reaction mixture of G-CSF and PEG-OPSS (5 kDa) in physiological condition at reaction time 0.5, 3.5, 28 h (**B**, **C**, **D**) using C4 VIDAC™ column.
Figure 2: SDS-PAGE analysis of G-CSF native *(lane 1*) and G-CSF PEG-OPSS conjugates in physiological conditions at reactions times 0.5, 2.5, 3.5, 6, 28, 51 h *(lane 2-7*).
Figure 3: : RP-HPLC chromatograms. G-CSF native (**A**); reaction mixture of G-CSF and PEG-VS (5 kDa) in physiological condition after 28 h of reaction (**B**) using C4 VIDAC™ column.
Figure 4: SDS-PAGE analysis of G-CSF native *(lane 1*) and G-CSF PEG-VS conjugates in physiological conditions at reactions times 0.5, 2.5, 3.5, 6; 28, 51 h *(lane 2- 7*).
Figure 5: Far UV-CD spectra of G-CSF native in standard condition and after denaturation-renaturation process by Guanidine HCl. The spectra were obtained at 25°C in acidic solution at pH 3.5.
Figure 6: RP-HPLC chromatograms using C4 VIDAC™ column. G-CSF native (**A**); reaction mixture of G-CSF and PEG-OPSS (5 kDa) in presence of 3M Urea (**B**); reaction mixture of G-CSF and PEG-OPSS (5 kDa) in presence of 2, 4, 6M guanidine HCl (**C**, **D**, **E**).
Figure 7: RP-HPLC chromatograms. G-CSF native (**A**); reaction mixture of G-CSF and PEG-VS (5 kDa) in presence of 2, 4, 6M guanidine HCl (**B**, **C**, **D**), using C4 VIDAC™ column.

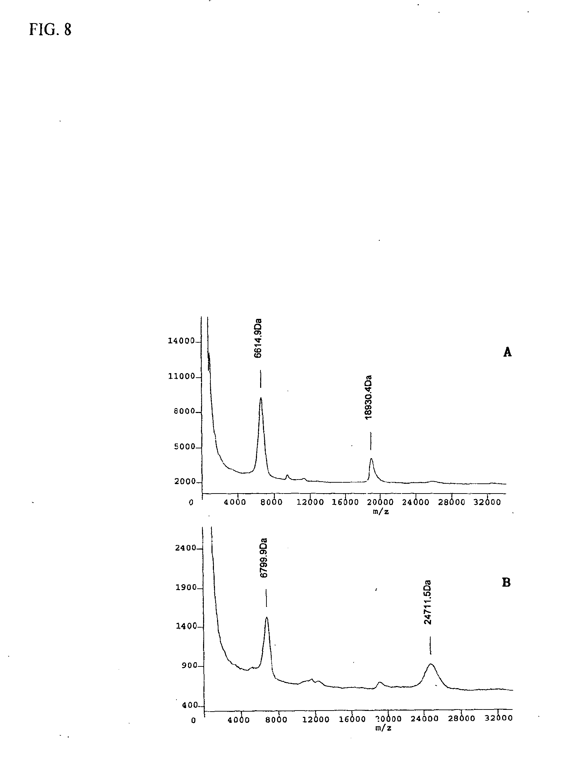

Figure 8: MALDI-TOF mass spectroscopy of G-CSF native (**A**); G-CSF-PEG-OPSS (5kDa) conjugate obtained from reaction mixture by RP-HPLC chromatography (**B**).

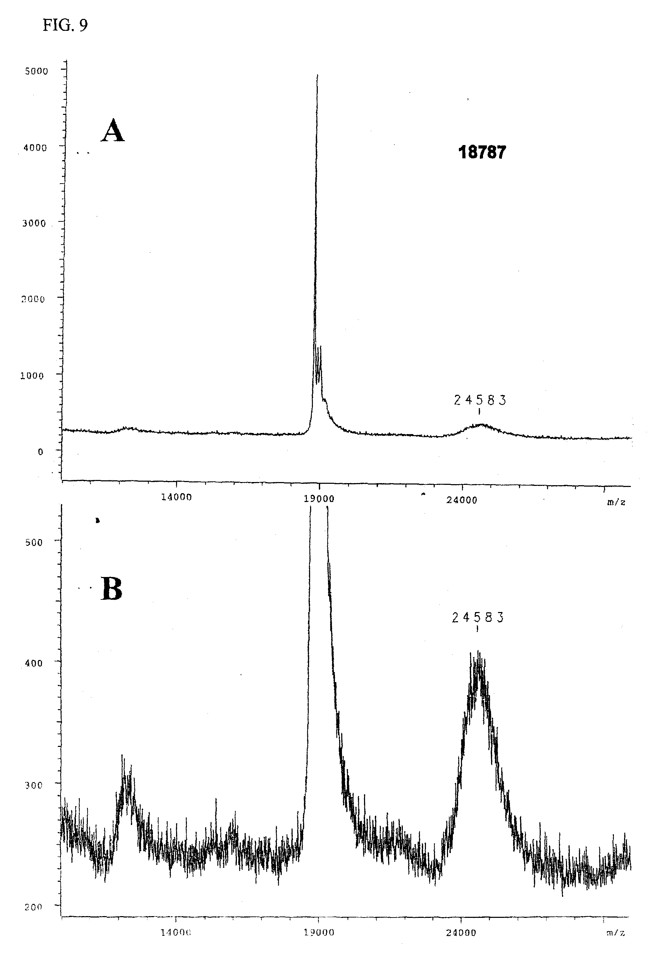

Figure 9: MALDI-TOF mass spectroscopy of the reaction mixture between G-CSF and PEG-VS (5kDa) (**A**). Spectrum expanded (**B**).

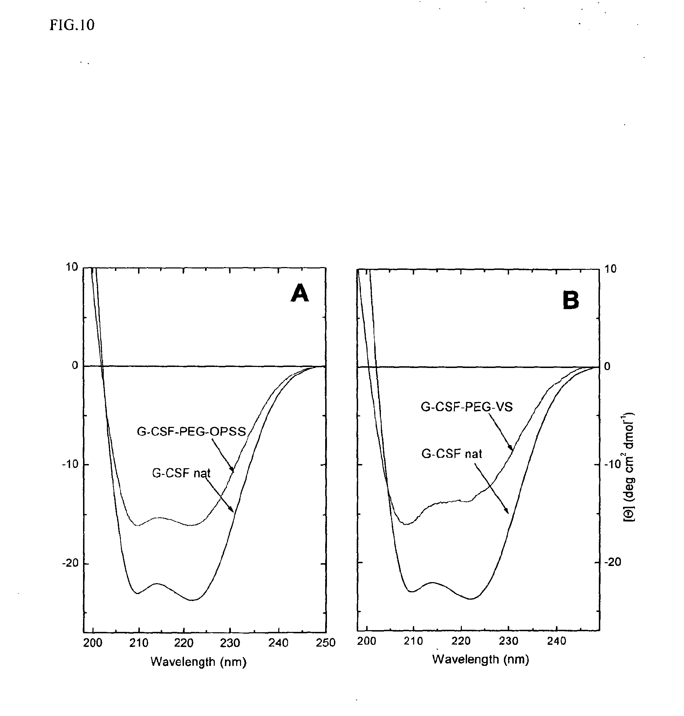

Figure 10: Far-UV CD spectra of G-CSF native and conjugated **A**, G-CSF-PEG-OPSS (5kDa). **B**, G-CSF-PEG-VS (5 kDa). Spectra were taken at 25°C in 10 mM ac. acetic, 0.004% tween, 10 mg/ml mannitol, pH 4.
Figure 11: Fluorescence emission spectra of G-CSF native, G-CSF-PEG-OPSS (5 kDa) and G-CSF-PEG-VS (5 kDa) conjugates. Spectra were taken exiting the samples (1.3 µM) at 295 nm, in presence of CsCl (**A**), Nal (**B**).
Figure 12: Evaluation of the stimulating effects of G-CSF native, G-CSF-PEG-OPSS, G-CSF-PEG-VS conjugates in the proliferation of NFS-60 cells. Responses were obtained by MTT colorimetric assay reading D.O. at 540 nm.
Figure 13: RP-HPLC chromatograms of reaction mixture of G-CSF and PEG-OPSS (10 kDa) using C4 Phenomenex™ column.
Figure 14: RP-HPLC chromatograms of reaction mixture of G-CSF and PEG-OPSS (20 kDa) using C4 Phenomenex™ column.
Figure 15: Far-UV CD spectra of G-CSF native and PEG-OPSS conjugates (5 kDa black, 10 kDa violet, 20 kDa green). Spectra were taken at 25°C in 10 mM ac. acetic, 0.004% tween, 10 mg/ml mannitol, pH 4.
Figure 16: RP-HPLC chromatograms of native rhG-CSF (fig.16A) and of the reaction mixture of G-CSF and PEG-Mal (20 kDa).

## Claims

1. A conjugate of PEG and human G-CSF or an analogue of G-CSF, **characterized in that** the cysteine thiol in position 17 of the human G-CSF or in the corresponding position of the G-CSF analogue is conjugated to a PEG molecule.

2. A conjugate according to claim 1 **characterised in that** the G-CSF has the following formula: 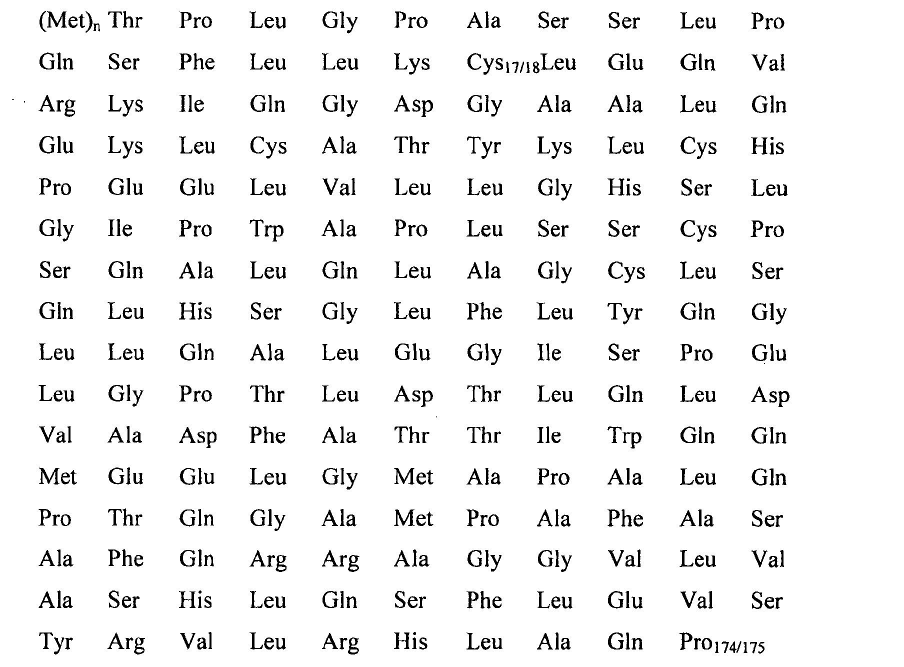 wherein n is 0 or 1.

3. The conjugate according to claim 1 or 2 where the PEG is linear or branched and is monofunctional or bifunctional.

4. The conjugate of claims from 1 to 3 where the PEG is bifunctional and comprises one molecule of polymer and 2 molecules of G-CSF or of its analogue.

5. The conjugates of any of the preceding claims where the PEG has MW ranging between 800 to 80.000 Da and preferably 5.000 to 40.000 Da.

6. Pharmaceutical composition comprising a conjugate according to any of the previous claims.

7. Use of a conjugate according to any of the previous claims for the manufacturing of a pharmaceutical formulation for the treatment of neutropenia (chronic, chemotherapy induced, HIV induced, bone marrow transplantation).

8. A process for the manufacture of a conjugate of a polymer and a polypeptide with a sterically hindered cysteine group comprising the following steps:
(i) subjecting the polypeptide to conditions inducing reversible denaturation of the polypeptide,
(ii) conjugation of the denatured polypeptide obtained in step (i) with a thiol reactive polymer, under denaturing conditions,
(iii) subjecting the conjugate obtained in step (ii) to conditions which promote renaturation of the conjugate and afford the desired conjugate.

9. The process according to claim 8 wherein the polypeptide is G-CSF or its analogue and the polymer is PEG.

10. The process of claim 8 wherein the reversible denaturation is obtained by addition of a suitable amount of denaturating agent selected among urea, guanidine chloride or isothiocianate, dimethil-urea, high neutral salt concentrations and organic solvents.

11. The process of claim 10 wherein the denaturating agent is Guanidine Chloride and the concentration of Guanidine Chloride is more than 1 M and is preferably 4 M to 6 M.

12. The process of claims 9-11 wherein the PEG has a thiol reactive group selected from OPSS, VS, N-maleimide or iodoacetamide.

13. The process of claims 8-12 where the renaturation is obtained removing the denaturant agent by dialysis, ultrafiltration, chromatography or dilution.

14. The process according to any of the preceding claims, **characterized by** being carried out in an aqueous buffered solution at pH of 5 to 11.

15. The process of claim 14, where the buffering agent is TRIS or phosphate buffer and the pH is around 7.

16. A process according to claims 8-15 **characterised in that** step (ii) is carried out at a temperature comprised between 5°C to 50°C , preferably at a temperature of between 20°C to 40°C.
